# EUROPÄISCHE PATENTANMELDUNG

(11) **EP 2 052 667 A1**
(43) Veröffentlichungstag der Anmeldung: **29.04.2009**
(21) Anmeldenummer: 08167075.4
(22) Anmeldetag: 21.10.2008
(51) Int. Cl.: A61B 1/00, A61B 1/018

(54) **Endoskop mit verschliessbarem Instrumentenkanal**

(30) Priorität: 25.10.2007 DE 202007014897 U
(71) Anmelder: Fritz GmbH, 78532 Tuttlingen (DE)
(72) Erfinder: Fritz, Rolf, 78532 Tuttlingen (DE)
(74) Vertreter: Fitzner, Uwe

(57) **Zusammenfassung**

Die vorliegende Erfindung betrifft ein Endoskop mit Instrumentenkanal, dessen Einfuhrschlauchspitze verschließbar ist.

## Beschreibung

Die vorliegende Erfindung betrifft ein Endoskop mit verschließbarem Instrumentenkanal.

Endoskope sind optische Systeme zur Visualisierung von Körperhöhlen und inneren Organen im medizinischen und veterinärmedizinischem Bereich. Dem gleichen Aufbau und Funktionsprinzip folgend aber doch zu unterscheiden sind sog. technische Endoskope, die für nicht einsehbare Hohlräume im Bereich der Technik eingesetzt werden.

Grundsätzlich unterscheidet man zwischen sog. starren und flexiblen Endoskopen. D. h. die optischen Systeme sind entweder in einem starren Rohr, oder in einem flexiblen meist steuerbaren Schlauch eingesetzt. Hierbei gibt es nach dem Stand der Technik auch Kombinationen beider Systeme. Beide Endoskoparten haben Vor- und Nachteile hinsichtlich der Produktion, der Pflege (Reinigung und Desinfektion) und des jeweiligen Einsatzgebietes am Menschen, Tier oder im Bereich der Technik.

Im medizinischen und veterinärmedizinischen Bereich werden vorzugsweise flexible Endoskope in der Regel für die Magen- und Darmuntersuchung (Gastroskopie) oder für die Atemwegsuntersuchung (Laryngoskopie oder Bronchoskopie) eingesetzt. Äußerlich unterscheidet man an einem flexiblen Endoskop im Allgemeinen einen sog. Einführschlauch, also ein Teil, welcher in dem Patienten eingeführt wird, von einem Kontroll-/Hauptteil mit dem Steuer- oder Triebrädern und dem Okular zum Einsehen sowie den sog. Versorgungsschlauch, der die Verbindung zur Lichtquelle und/oder Video/Licht-Steuereinheit herstellt.

Verschiedene Ausführungsformen von Endoskopen sind beispielsweise in der DE 34 17 571, 392343 A1, G8916160.2, DE 39 23 243 C2, DE 39 23 243 A1 beschrieben.

Bei Einsatz der Endoskope findet neben der Visualisierung des entsprechenden Organs in vielen Fällen eine Gewebeprobenentnahme (Biopsie) statt. Dazu ist im flexiblen Endoskop (Gastroskop), Bronchoskop, (Laryngoskop) ein sog. Arbeitskanal und auch ein Instrumentenkanal eingebaut, durch den ein Arbeitsinstrument, z. B. ein Biopsieinstrument zur Gewebeprobenentnahme eingeführt werden kann. Mehrere Kanäle parallel sind möglich. Über diesen Kanal kann auch eine Absaugung erfolgen.

Über einen oder mehrere Kanäle wird eine Spülung oder Luftsufflation durchgeführt, um das Frontlinsensystem bzw. das Gesichtsfeld zu reinigen bzw. das Organ aufzudehnen. Üblicherweise sind im Haupt-/Kontrollteil die Kanäle durch Ventile zum Öffnen und Schließen unterbrochen. Der Kanal, der Instrumente führen soll (der Kanal mit dem größten Innendurchmesser), hat am Hauptteil aus praktischen Gründen seinen Eingang. Er wird über eine Y-Verzweigung im Hauptteil als Absaugkanal geführt und findet im Versorgungsschlauch des Endoskops Platz. Ein Ventil kann die Absaugung im Hauptteil entsprechend steuern.

Zum Verschließen und Unterbrechen werden verschiedene Systeme vorgeschlagen. Ein Beispiel ist in der DE 199 11 911 A1 beschrieben. Die bisher üblichen Ventile verschließen und unterbrechen den Durchgang an einer beliebigen Stelle am Haupt- oder Versorgungsteil des Endoskops.
Diese Systeme haben aber den Nachteil, dass der Kanal an seinem Ende offen bleibt für Schleim, Blut und Infektionskeime, die beim Einführen in das Kanalende bis zur Ventilstelle hinein gedrückt werden. Infolgedessen muss bei der Reinigung und Desinfektion immer sorgsam darauf geachtet werden, dass alle Kanalanteile berücksichtigt werden, was bei Durchmessern von 0,1 mm bis 3,5 mm auf Längen von 3.000 bis 6.000 cm u. U. Schwierigkeiten bereiten kann. Es können sich im feuchten Kanalmilieu daher sehr leicht desinfektions-resistente Bakterienrasen bilden, die auch ein hygienisches Problem darstellen. Zusätzlich bleiben Schmutzteile auf der vergrößerten Oberfläche der Endoskopspitze hängen. Deshalb wird ein Kanal zur Linsenreinigung benötigt, was die Oberfläche weiter erhöht und den Gesamtdurchmesser des Systems vergrößert.

Die vorliegende Erfindung hat sich nunmehr die Aufgabe gestellt, die aufgeführten Nachteile des Standes der Technik zu vermeiden.

Diese Aufgabe wird durch ein Endoskop mit Instrumentenkanal gelöst, dessen Einführschlauchspitze verschließbar ist.

Um dieses Ziel zu erreichen, wird erfindungsgemäß an der Einführschlauchspitze vorzugsweise ein Verschlusselement angeordnet. Dieses Element kann beliebige Ausgestaltung haben. So können Lichtstopfen mittels einer Spiralfeder am Ende des Arbeitskanals angeordnet sein. Ebenso kann eine federnd gelagerte Fixierschraube an der Öffnung des Kanals angebracht sein, so dass dieser mittels einer Spannmutter verschließbar ist. Ebenso können aber auch andere aus dem Stand der Technik bekannte Verschlusssysteme eingesetzt werden. Hierzu gehören Ventilklappen oder auch das System gemäß der DE 199 11 911 A1. Der Verschlussmechanismus arbeitet in einer bevorzugten Ausführungsform im Prinzip wie ein Kanaldeckel. Man schiebt von der (Einführspitze) her diesen "Deckel", der an einem gefederten Spiraldraht hängt, in den Kanal und verspannt das Ganze am Ende (Eingang des Instrumentierkanales). So wird der Verschluss permanent unter einer gewissen Schließspannung gehalten.

Erfindungswesentlich ist, dass die Ventile oder andere Verschlusssysteme derart am Ende der Einführschlauchspitze angeordnet sind, dass kein Kanalabschnitt mehr offen ist für das Eindringen von Schleim, Blut und Infektionskeimen. Zu diesem Zweck ist die Einfuhrschlauchspitze erfindungsgemäß vorzugsweise bündig und wasserdicht und keimdicht verschließbar. Dadurch wird jegliches Eindringen von Keimen und Schmutzteilen verhindert. Eine Desinfektion und Reinigung des Instrumentenkanals oder von Abschnitten des Kanals kann damit nach Gebrauch entfallen. Auch kann erfindungsgemäß auf einen zusätzlichen Kanal zur Spülung und Linsenreinigung und Insuflation verzichtet werden. Die erfindungsgemäße Anordnung einer verschließbaren Einführschlauchspitze bietet mithin folgende wesentliche Vorteile:
- Kein Eindringen von Keimen und Schmutzteilen durch einen wasserdichten Verschluss.
- Eine Reinigung und Desinfektion des Instrumentenkanals kann bei Nichtgebrauch entfallen.
- Die Anwenderfreundlichkeit wird erhöht und das Übertragungsrisiko von Infektionen gleichzeitig gesenkt.
- Auf einen zusätzlichen Kanal zur Spülung und Linsenreinigung und Insufflation kann verzichtet werden, da sich Schleimpartikel am Distalende im geringeren Umfang festsetzen.

Die Möglichkeit des Verzichts auf einen zusätzlichen Kanal (Spül/Insuflationskanal) führt außerdem zu einem kleineren Gesamt-/Außendurchmesser des Einführteils, was die Anwendung erheblich erleichtert. Dies ist vor allen Dingen im Veterinärbereich von Bedeutung, da kleinere Durchmesser die Akzeptanz des zu untersuchenden Tieres verbessern. Die fehlende Sprühdüse vereinfacht die Konstruktion und damit die Produktionskosten. Auch dadurch wird die Anwenderfreundlichkeit erhöht.

Der größte Vorteil des erfindungsgemäßen Systems liegt in der Möglichkeit einer schnellen und unkomplizierten Reinigung der Endoskope. Dies gilt besonders für eine mobile Außenpraxis. Denn hier kosten aufwändige Reinigungsverfahren viel Zeit, sofern deren Durchführung überhaupt machbar ist. Die Keimverschleppung durch schlecht desinfizierte Endoskope ist bei Einsatz des erfindungsgemäßen Systems um ein Vielfaches herabgesetzt. Sowohl für den Anwender als auch den Hersteller stellt dieses System mithin eine deutliche Verbesserung im Hinblick auf die verbesserte Einsetzbarkeit des Gerätes dar. Ebenso sind die Produktionskosten gegenüber dem bisherigen Stand der Technik gesenkt.

Das erfindungsgemäße System kann sowohl bei starren als auch bei flexiblen Endoskopschläuchen eingesetzt werden. Der besondere Vorteil des erfindungsgemäßen Systems zeigt sich vor allen Dingen bei Einsatz von flexiblen Endoskopen für die Magen- und Darmuntersuchung sowie für die Atemwegsuntersuchung.

Im Folgenden wird die Erfindung unter Bezugnahme auf die Figuren näher beschrieben:

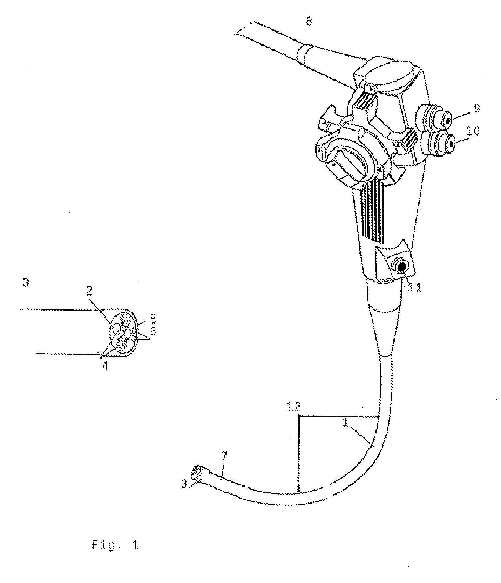

In Figur 1 ist ein standardmäßiges Endoskop nach dem Stand der Technik dargestellt. Dieses System umfasst einen flexiblen Schlauch 1, in welchem ein sog. Arbeitskanal oder Instrumentenkanal 2 eingesetzt ist. In dem Distalende 3 sind Lichtleiter 4, Objektiv 5 und Luft-/Wasserdüsen 6 angeordnet. Das Distalende ist hinter dem Abwickelungsteil 7 angeordnet. In dem Kontrollteil 8 sind u. a. das Absaugventil 9, das Luft-/Wasserventil 10 sowie die Kanalöffnung 11 angeordnet. Über das Absaugventil 9 lässt sich die Absaugung auslösen. Über das Luft/Wasserventil wird die Spülung der Luft-/Wasserdüsen 6 gesteuert. Über die Kanalöffnung 11 kann somit eine Spülung oder Luftisuflation geführt werden, um das Frontlinsensystem bzw. das Gesichtsfeld zu reinigen bzw. das Organ aufzudehnen. Der Kanal, der die Instrumente führen soll (der Kanal mit dem größten Innendurchmeser) hat an dem Kontrollteil 8 seinen Eingang bzw. die Kanalöffnung 11. Er wird über eine Y-Verzweigung im Kontrollteil 8 als Absaugkanal fortgeführt und findet im Versorgungsschlauch 12 des Endoskops Platz. Entsprechend wird die Absaugung im Hauptteil mittels des Absaugventils 9 gesteuert.

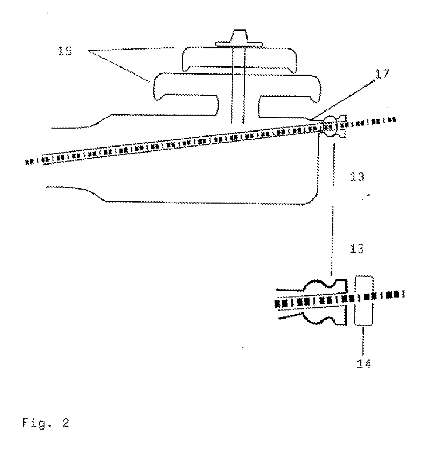

In Figur 2 ist ein verschließbarer Arbeitskanal 17 für flexible Endoskope gemäß der vorliegenden Erfindung dargestellt. An dem Ende 13 des Arbeitskanals 17 ist eine federnd gelagerte Fixierschraube oder Spannmutter 14 angeordnet. Diese können mittels der Triebräder 15 betätigt werden.

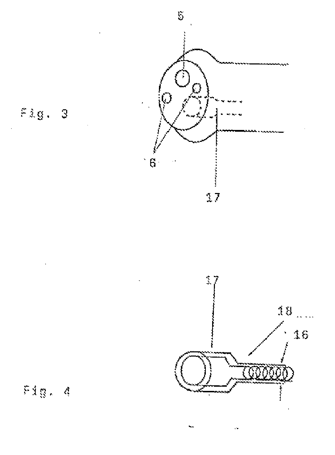

In der Figur 3 ist das Ende des Arbeitskanals mit Linse 5 und Lichtleiter 6 dargestellt.
In der Figur 4 ist ein Verschluss mit Dichtstopfen 18 in dem Arbeitskanal 17 abgebildet. Über den Spiraldraht 16 wird der Dichtstopfen unter Spannung gehalten.

## Patentansprüche

1. Endoskop mit Instrumentenkanal (2), dessen Einfuhrschlauchspitze (13) verschließbar ist.

2. Endoskop nach Anspruch 1 **dadurch gekennzeichnet,**
**dass** an der Einfuhrschlauchspitze (13) ein Verschlusselement (14) angeordnet ist.

3. Endoskop nach einem der vorhergehenden Ansprüche **dadurch gekennzeichnet,**
**dass** das Verschlusselement (14) bündig und wasserdicht ausgestaltet ist.

4. Endoskop nach einem der vorhergehenden Ansprüche **dadurch gekennzeichnet,**
**dass** der Instrumentenkanal (2) starr ist.

5. Endoskop nach einem der vorhergehenden Ansprüche **dadurch gekennzeichnet,**
**dass** der Instrumentenkanal (2) flexibel ist.

6. Verwendung des Endoskops nach einem der vorhergehenden Ansprüche für die Magen-/Darm-Untersuchung oder die Atemwegsuntersuchung.
